# EUROPEAN PATENT SPECIFICATION

(11) **EP 0 592 378 B1**
(45) Date of publication and mention of the grant of the patent: **08.05.1996**
(21) Application number: 93830410.2
(22) Date of filing: 07.10.1993
(51) Int. Cl.: A61B 17/04, A61B 17/11

(54) **An instrument for performing purse-string sutures**
Vorrichtung für Nähte mit Beutel-Schnur-Struktur
Instrument pour sutures en cordon de bourse

(30) Priority: 08.10.1992 IT MI922320
(43) Date of publication of application: 13.04.1994
(73) Proprietor: Rebuffat, Carlo, Milano (IT); Rosati, Riccardo, Milano (IT)
(72) Inventor: Rebuffat, Carlo, Milano (IT); Rosati, Riccardo, Milano (IT)
(74) Representative: Pizzoli, Pasquale Vincenzo

(56) References cited:
- EP-A- 0 332 591

## Description

This invention relates to an automatic instrument for performing the so-called purse-string sutures, for surgical use, including two jaws carrying teeth, making it possible to use the instrument in narrw spaces, and at depth, without having to manually insert the needles into said jaws.

As it is already known, in order to be able to use in a suitable way the mechanical circular suturing instruments which have been introduced in the surgical practice long since, it has become necessary to resort to the so-called purse-string sutures.

This type of suture does not pose any particular problem, and it is quite fast to perform, provided the operation doesn't have to be performed in narrow, deep spots. In the latter condition, these sutures may be performed manually or mechanically by means of straight jaws with teeth, provided with openings through which the needles, or a single needle, are manually slid, first along one jaw in a certain direction, and then along the other jaw in the opposite direction. It is a drawback of said instrument, as it is disclosed in British Patent No. 2 081 099, that it can be used only in wide operating areas, where it is possible to insert the instrument and to insert the straight needle into the special jaw openings.

In order to overcome said problems, an instrument has been devised which is provided with two arc shaped jaws, supported by correspondingly arc shaped arms. This instrument, as it is disclosed in European Patent No. 0-119967, while being improved compared to the instrument disclosed in British Patent No. 2 081 099, does not thouroughly overcome all the problems pertaining to the purse-string suture technique: in fact, in this case as well, the needles being used have to be inserted manually and that operation may prove difficult to perform in the narrowest surgical spaces.

Obviously, a wide surgical space is required for a large needle length, therefore the technical problem to be solved has been that of providing an automatic short needle instrument, in order to be able to use such an instrument in narrow spots.

Such a technical problem has been overcome by an automatic instrument as claimed in European Patent 0 332 591, owned by the Assignee of this invention, and which discloses an instrument including in combination the features of the pre-characterizing portion of claim 1.

This instrument features the possibility to be used with reduced length needles, in particular a length no shorter than the length of the recesses between adjacent teeth of the instrument jaws.

The object of this invention has been to provide an instrument for performing purse-string sutures to be used with long needles in tight spots, which is simple and reliable in operation and has a low manufacturing cost.

Also it should be made possible to perform a partial closure of the anatomical tissue to be sutured, for a correct positioning thereof, followed by a final closure in order to clamp the tissue in a non-removable way.

The features and advantages listed herein above, which are all met by the instrument as specified in claim 1, will become apparent from the following detailed description of a unique preferred embodiment of the instrument, which is shown for exemplary and non limiting purposes only in the attached drawings, wherein:
Figure 1 shows the instrument in a longitudinal section;
Figure 2 is a perspective view of the instrument of Figure 1;
Figure 3 is an enlarged partial sectional view of the instrument, along line III-III of Figure 1;
Figure 4 is a view of the instrument, with closed jaws looking in the direction of arrow A in Figure 2;
Figure 5 is a view of the instrument with open jaws, looking in the direction of arrow B in Figure 2; and
Figure 6 is a cross-sectional view of both jaws along line VI-VI of Figure 1.

Referring now to the attached drawings, the instrument includes substantially a pair of jaws 1 and 2, carrying teeth, and integral with a pair of supports 3 and 4. The latter of which carries a drum 5 provided with an actuating member comprising a knob 6. Jaws 1 and 2 are provided with teeth 7 and 8 respectively which, as it can be clearly seen in Figure 6, are provided with openings 9 and 10 having side slots 11. Long and flexible needles 12 and 13 slide along said openings 9 and 10, as shown in Figure 3, carrying the ends of a suture thread 14 fastened to the tip thereof.

According to the subject of this invention, a hollow pusher member , comprising a small gauge flexible tube 15, holds said needles 12 and 13 and suture thread 14.

Precisely, small gauge tube 15 acts as a hollow pusher member for needles 12 and 13, since it carries a inner gauge tube 16 therewithin, comprising an annular shoulder 17 engaging needles 12 and 13. Suture thread 14 is held inside inner gauge tube 16, whereby through a displacement of small gauge tube 15 in the direction of arrow C, the needles are dragged by said annular shoulder along openings 9 and 10 of teeth 7 and 8.

The pusher member comprised of small gauge tube 15, holding the needles and suture thread therewithin, is thus connected at one end to drum 5, while the other end thereof is connected to the needles.

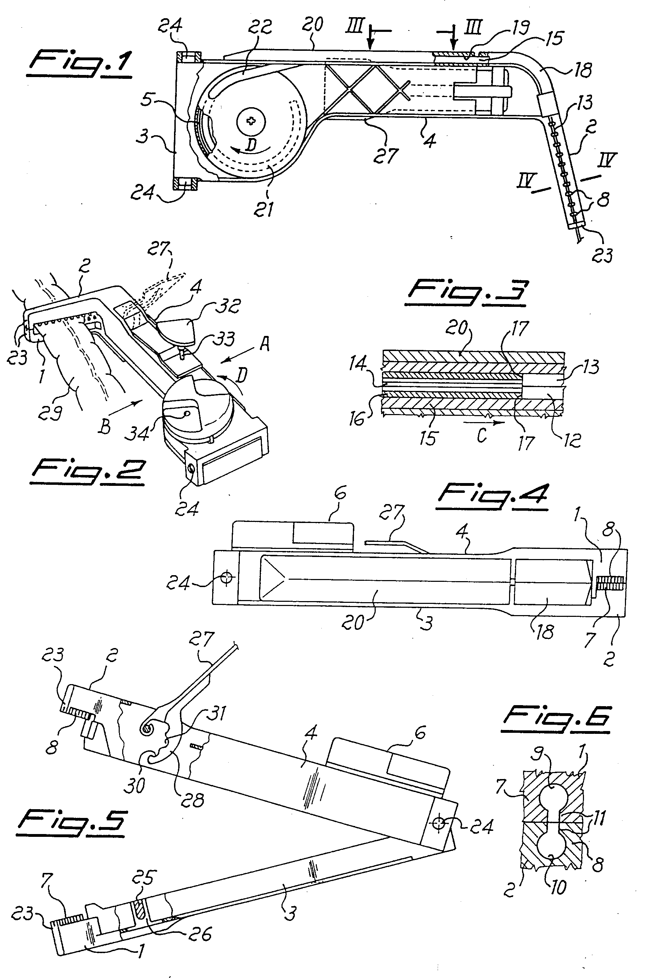

It should be noted that jaws 1 and 2 form an angle with respective supports 3 and 4, in order to enable the instrument to be more easily introduced in narrow spaces, and that an arc shaped transition piece 18 is provided between the jaws and their supports, whereby needles 12 and 13, being flexible, under the push of small gauge tube 15, follow an arc shaped path at said transition piece 18. Small gauge tube 15 extends further according to a straight path along a narrow passage 19 towards drum 5. As it may be seen in Figure 1, said narrow passage 19 slidingly carrying small gauge tube 15, is provided within the thickness of cover 20 fastened to support 4, but it might as well be obtained by molding within said support 4.

As it is shown in Figure 1, drum 5 within support 4 is provided with circumferential slot 21, preferably extending along an angle of 270°, said slot holding small gauge tube 15 coiled therein. The latter is guided by a short fixed slot 22 provided within support 4 and connected to slot 21 provided inside drum 5 rotating for three quarters of a round angle by means of knob 6, in the direction of arrow D, shown in Figure 1. In this way, said small gauge flexible tube 15 is unrolled from said circumferential slot 21 and is made to slide along narrow passage 19 in order to push needles 12 and 13 with the suture thread 14 along openings 10 and 11 of jaws 1 and 2 all the way to make them to project out of the free ends 23 of said jaws 1 and 2. Since the jaws are straight the needles as well are straight, long and flexible, in that they are automatically pushed by inner gauge tube 16 along said arc shaped transition piece 18 and through the entire length of jaws 1 and 2.

The needles being long, it is positive that they regularly run straight into the jaw openings 10 and 11.

As it is shown in Figures 4 and 5, for opening and closing jaws 1 and 2, supports 3 and 4 overlying each other are hingedly connected at 24 and they are respectively provided with a small support 25 having an opening 26, and with an operating lever 27 having a hook 28.

For closing the two jaws 1 and 2, lever 27 is actuated in such a way that starting from the position shown in Figure 5 the two jaws are brought together and hook 28 is inserted into opening 26 of small support 25.

By a reverse operation of lever 27, starting from the closed position of Figure 4, supports 3 and 4 and thereby jaws 1 and 2 may be opened all the way to reach the position shown in Figure 5. As it is known, by opening the jaws it is possible to insert anatomical tissue 29 to be sutured, between the jaws, and thereafter the jaws are closed.

According to a further feature of this invention, hook 28 is provided with a pair of notches 30 and 31, the first notch being adapted to get into engagement with said opening 26 for a partial closure of jaws 1 and 2, whereby anatomical tissue 29 may be displaced in order to be correctly positioned before performing the suture.

The second notch is brought into engagement with said opening 26 when closing jaws 1 and 2 tight, whereby anatomical tissue 29 to be sutured gets firmly clamped therebetween.

Suture thread 14 has a diameter smaller than the width of slots 11, which are narrower than the diameter of openings 9 and 10 where the needles pass, whereby suture thread 14 may come out of slots 11 when it is pulled by needles 12 and 13, which slide along openings 9 and 10 of teeth 7 and 8, without being able to come out through slots 11.

In order to prevent knob 6 from being unintentionally rotated there has been provided a sector 32 carrying a peg 33 which is inserted in a hole 34 of drum 5. Only after said sector has been pulled out of knob 6, as it is shown in Figure 2, knob 6 is allowed to freely rotate in the direction of arrow D in order to displace said pusher member or small gauge tube 15 against needles 12 and 13 to make them to project out of the free ends 23 of jaws 1 and 2.

Practical-applicational type modifications may be made to the embodimental details of this invention without exceeding the scope of the inventive idea, as it is claimed in the following.

## Claims

1. An instrument for purse-string sutures for surgical use, including two jaws (1, 2) carrying teeth (7, 8) provided with openings (9, 10) having a side slot (11) and two needles, said openings (9, 10) being adapted for through passage of the needles (12, 13) therealong, said needles carrying the ends of a suture thread (14) fastened thereto, said jaws (1, 2) being integral with supports (3, 4) adapted to be opened in order to insert anatomical tissue (29) to be sutured between said jaws, characterized in that one of said supports (3, 4) carries a pusher member (15) which is connected at one end to an actuating member (6) while at the opposite end it encloses the two needles (12, 13) and the suture thread (14) so that by operating said actuating member (6), needles (12, 13) are pushed by said pusher member (15), the needles being long and flexible so that they are respectively urged along said openings (9, 10) of jaws (1, 2) all the way to make needles (12, 13) to project out of free ends (23) of said jaws (1, 2).

2. The instrument of Claim 1, characterized in that said actuating member comprising a knob (6) is integral with a drum (5) rotatingly mounted on support (4) and in that the pusher member comprising a small gauge tube (15) is wrapped around said drum (5) when knob (6) is at rest, while it unrolls on actuation of said knob (6) adapted to move said gauge tube towards jaws (1, 2) whereby needles (12, 13) are displaced along openings (9 and 10) of jaws (1, 2).

3. The instrument of Claim 2, characterized in that said drum (5) is provided with a circumferential slot (21) wherein small gauge tube (15) is wrapped and which is connected to a short slot (22) integral with support (4), within which there slides small gauge tube (15) while unrolling out of circumferential slot (21) due to the rotation of knob (6) in the unrolling direction.

4. The instrument of Claim 4, characterized in that on the upper portion of support (4) carrying knob (6) there is provided a passage (19) which extends from the short slot (22) through which small gauge tube (15) is led by means of said knob (6), all the way to the starting position of jaws (1, 2).

5. The instrument of Claim 5, characterized in that said thin channel (19) wherein small gauge tube (15) slides may be managed within the thickness of a cover (20) which closes support (4), or it may be molded on said support (4), or else attached to said support in the form of a small gauge tube.

6. The instrument of Claim 2, characterized in that small gauge tube (15) is wrapped, at one end thereof, around said drum (5) while at the opposite end it carries an inner annular shoulder (17) in engagement with said needles (12, 13) to which the ends of suture thread (14) are fastened, said thread benig able to go through the opening defined by the circular shoulder (17).

7. The instrument of Claim 7, characterized in that said small gauge tube (15) is provided with another inner small gauge tube (16) whose end on the side of needles (12, 13) provides said annular shoulder (17).

8. The instrument of Claim 1, characterized in that said supports (3, 4) are hingedly connected to each other (24) at their end remote from jaws (1, 2) and in that one of said supports (3, 4) is provided with a lever (27) actuatable from outside, and provided with a hook means (28), while the other support member is provided with an opening (26) of a small support (25), into which said hook means (28) gets inserted for closing said jaws (1, 2), while if said hook means is set free from said opening (26), the pair of supports (3, 4) and related jaws (1, 2) can be opened in order to introduce therebetween anatomical tissue (29) to be sutured.

9. The instrument of Claim 9, characterized in that said hook (28) is provided with a pair of notches (30, 31), first notch (30) being adapted to get engaged into said opening (26) for a partial closure of said jaws (1, 2) so that the anatomical tissue to be sutured may still be displaced to find the correct position thereof, and second notch (31) is adapted to engage with said opening (26) for completely closing jaws (1, 2).

10. The instrument of Claim 2, characterized in that a sector (32) provided with a peg (33) inserted into a bore (34) provided in said drum (5) is adapted to be pulled out of said bore (34) in order to enable said knob (6) to rotate freely to displace said small gauge tube (15) and said needles (12, 13) therewith, along jaws (1, 2).

11. The instrument of Claim 4, characterized in that circumferential slot (21) of drum (5) has such a diameter that to a three quarter of a turn rotation of said knob (6) there corresponds a forward motion of said small gauge tube (15) that makes needles (12, 13) to project out of free end (23) of said jaws (1, 2).

## Patentansprüche

1. Instrument für Beutelschnurnähte für chirurgische Zwecke, mit zwei Backen (1,2), die Zähne (7,8) tragen, die mit Öffnungen (9,10) versehen sind, die einen Seitenschlitz (11) aufweisen und zwei Nadeln, welche Öffnungen (9,10) ausgebildet sind für den Durchgang der Nadeln (12,13), welche Nadeln die Enden von Nahtfäden (14) tragen, die an den Nadeln befestigt sind, welche Backen (1,2) einstückig mit Trägern (3,4) ausgebildet sind, die geöffnet werden können zum Einfügen eines anatomischen Gewebes (29), das zwischen den Backen zu vernähen ist, dadurch **gekennzeichnet**, daß einer der Träger (3,4) ein Schubglied (15) trägt, das an einem Ende an einem Befestigungsglied befestigt ist, während es am anderen Ende die beiden Nadeln (12,13) und den Nahtfaden (14) einschließt, so daß durch Betätigung des Betätigungsgliedes (6) die Nadeln (12,13) durch das Schubglied (15) vorgeschoben werden, wobei die Nadeln lang und flexibel sind, so daß sie durch die Öffnungen (9,10) der Backen (1,2) über die gesamte Strecke vorgeschoben werden, so daß die Nadeln (12,13) aus den freien Enden (23) der Backen (1,2) hinausragen.

2. Instrument nach Anspruch 1, dadurch **gekennzeichnet**, daß das Betätigungsglied einen Knopf (6) umfaßt und einstückig mit einer Trommel (5) ausgebildet ist, die drehbar an dem Träger (4) angeordnet ist, und daß das Schubglied eine kleine Meßröhre (15) umfaßt und um die Trommel (5) herumgewickelt ist, wenn der Knopf (6) in Ruhe ist, während sie abgewickelt wird bei Betätigung des Knopfes (6) zur Bewegung der Meßröhre in Richtung der Backen (1,2), wobei die Nadeln (12,13) entlang den Öffnungen (9 und 10) der Backen (1,2) verschoben werden,

3. Instrument nach Anspruch 2, dadurch **gekennzeichnet**, daß die Trommel (5) mit einem Umfangsschlitz (21) versehen ist, in den die kleine Meßröhre (15) gewickelt ist und der verbunden ist mit einem kurzen Schlitz (22), der einstückig mit dem Träger (4) ausgebildet ist, in welchem Schlitz die kleine Meßröhre (15) gleitet, während sie sich aus dem Umfangsschlitz (21) bei Drehung des Knopfes (6) in Abrollrichtung abrollt.

4. Instrument nach Anspruch 4, dadurch **gekennzeichnet**, daß auf dem oberen Bereich des Trägers (4), der den Knopf (6) trägt, ein Kanal (19) vorgesehen ist, der sich von dem kurzen Schlitz (22) erstreckt, durch den die kleine Meßröhre (15) mit Hilfe des Knopfes (6) über den gesamten Weg zur Startposition der Backen (1,2) geführt wird.

5. Instrument nach Anspruch 5, dadurch **gekennzeichnet**, daß der dünne Kanal (19), in dem die kleine Meßröhre (15) gleitet, eingearbeitet ist in die Dicke einer Abdeckung (20), die den Träger (4) verschließt, oder eingeformt ist in den Träger (4) oder anderweitig an dem Träger in der Form einer kleinen Meßröhre angebracht ist.

6. Instrument nach Anspruch 2, dadurch **gekennzeichnet**, daß die kleine Meßröhre (15) an einem Ende um die Trommel (5) gewickelt ist, während sie am entgegengesetzten Ende eine innere ringförmige Schulter (17) in Eingriff mit den Nadeln (12,13) trägt, an welchen die Enden des Nahtfadens befestigt sind, welcher Faden in der Lage ist, durch die Öffnung hindurchzugehen, die durch die kreisförmige Schulter (17) gebildet wird.

7. Instrument nach Anspruch 7, dadurch **gekennzeichnet**, daß die kleine Meßröhre (15) mit einer weiteren inneren kleinen Meßröhre (16) versehen ist, deren Ende an der Seite der Nadeln (12,13) mit der ringförmigen Schulter (17) versehen ist.

8. Instrument nach Anspruch 1, dadurch **gekennzeichnet**, daß die Träger (3,4) miteinander (24) an den Enden, die den Backen (1,2) gegenüberliegen, scharnierförmig miteinander verbunden sind, und daß einer der Träger (3,4) mit einem Hebel (27) versehen ist, der von außen betätigbar ist, und mit einer Hakeneinrichtung (28) versehen ist, während der anderen Träger mit einer Öffnung (26) einer kleiner Abstützung (25) versehen ist, in welche die Hakeneinrichtung (21) eingefügt wird zum Schließen der Backen (1,2), während, wenn die Hakeneinrichtung aus der Öffnung (26) frei ist, die beiden Träger (3,4) und die entsprechenden Backen (1,2) geöffnet werden können zum Einfügen eines anatomischen Gewebes (29), das zu vernähen ist.

9. Instrument nach Anspruch 9, dadurch **gekennzeichnet**, daß der Haken (28) mit zwei Nuten (30,31) versehen ist, welche erste Nut (30) in Eingriff treten kann mit der Öffnung (26) zum teilweisen Schließen der Backen (1,2), so daß das anatomische Gewebe, das zu vernähen ist, noch verschiebbar ist zum Auffinden der korrekten Position, und daß die zweite Nut (31) vorgesehen ist zum Eingreifen in die Öffnung (26) zum vollständigen Schließen der Backen (1,2).

10. Instrument nach Anspruch 2, dadurch **gekennzeichnet**, daß ein Sektor (32), der mit einem Zapfen (33) versehen ist, in eine Bohrung (34) eingefügt ist, die sich in der Trommel (5) befindet, und daß der Sektor vorgesehen ist zum Herausziehen aus der Bohrung (34), damit der Knopf (6) freigegeben wird zur freien Drehung zur Verschiebung der kleinen Meßröhre (15) und der Nadeln (12,13) mit der Röhre entlang den Backen (1,2).

11. Instrument nach Anspruch 4, dadurch **gekennzeichnet**, daß der Umfangsschlitz (21) der Trommel (5) einen Durchmesser aufweist, daß eine dreiviertel Umdrehung des Knopfes (6) einer Vorwärtsbewegung der kleinen Meßröhre (12) entspricht, die die Nadeln (12,13) aus dem freien Ende (23) der Backen (1,2) austreten läßt.

## Revendications

1. Instrument pour sutures en cordon de bourse à fin chirurgicale, comportant deux mâchoires (1,2) ayant des dents (7,8) munies d'ouvertures (9,10) ayant une fente latérale (11) et deux aiguilles , les ouvertures (9, 10) étant adaptées pour être traversées par les aiguilles (12,13), sur leur longueur, ces aiguilles portant les extrémités d'un fil de suture (14) qui leur est fixé, ces mâchoires (1, 2) étant solidaires de supports (3, 4) pouvant être ouverts pour permettre l'insertion entre les mâchoires du tissu anatomique (29) à suturer, caractérisé en ce que l'un des supports (3, 4) porte un élément de poussée (15) dont l'une des extrémités est reliée à un élément de manoeuvre (6) alors que son autre extrémité enferme les deux aiguilles (12, 13) et le fil de suture (14) de sorte qu'en actionnant l'élément de manoeuvre (6), les aiguilles (12, 13) sont poussées par l'élément de pousée (15), les aiguilles étant longues et flexibles de sorte qu'elles sont sollicitées respectivement le long des ouvertures (9, 10) des mâchoires (1, 2), sur toute leur longueur, pour que les aiguilles (12 13) fassent saillie à l'extérieur des extrémités libres des mâchoires (1, 2).

2. Instrument selon la revendication 1, caractérisé en ce que l'élément de manoeuvre comprend un bouton (6) qui est solidaire d'un tambour (5) monté à rotation sur le support (4) et en ce que l'élément de poussée comprend un petit tube de calibrage (15) qui est enroulé autour du tambour (5) quand le bouton est au repos alors qu'il s'en déroule lors d'une manoeuvre du bouton (6) adaptée pour déplacer ce tube de calibrage vers les mâchoires (1-2) grâce à quoi les aiguilles (12-13) sont déplacées le long des ouvertures (9 et 10) des mâchoires (1-2).

3. Instrument selon la revendication 2,
caractérisé en ce que le tambour (5) est muni d'une fente circonférentielle (21) dans laquelle le tube de calibrage (15) est enroulé et qui est reliée à une courte fente (22) du support (4), à l'intérieur de laquelle glisse le petit tube de calibrage (15) tout en se déroulant de la fente circonférentielle (21) par suite de la rotation du bouton (6) dans le sens de déroulement.

4. Instrument selon la revendication 3,
caractérisé en ce que, sur la portion supérieure du support (4) portant le bouton (6), est prévu un passage (19) qui s'étend depuis la fente courte (22) et à travers lequel le petit tube de calibrage (15) est amené au moyen du bouton (6), sur toute la longueur à la position de départ des mâchoires (1-2).

5. Instrument selon la revendication 4,
caractérisé en ce que le conduit mince (19) dans lequel le tube de calibrage (15) coulisse peut être prévu à l'intérieur de l'épaisseur d'un couvercle (20) qui ferme le support (4) ou peut être moulé sur ce support (4) ou fixé d'une autre manière sur le support sous la forme d'un petit tube de calibrage.

6. Instrument selon la revendication 2,
caractérisé en ce que le petit tube de calibrage (15) est enroulé, à l'une de ses extrémités, autour du tambour (5) alors qu'à son autre extrémité il porte un épaulement tubulaire intérieur (17) au contact avec des aiguilles (12-13) auxquelles les extrémités du fil de suture (14) sont fixées, ce fil étant capable de traverser l'ouverture définie par l'épaulement circulaire (17).

7. Instrument selon la revendication 6,
caractérisé en ce que le petit tube de calibrage (15) est muni d'un autre petit tube de calibrage intérieur (16) dont l'extrémité située du côté des aiguilles (12-13) constitue l'épaulement annulaire (17).

8. Instrument selon la revendication 1,
caractérisé en ce que les supports (3-4) sont articulés l'un à l'autre à leur extrémité éloignée des mâchoires (1-2) et en ce que l'un des supports (3-4) est muni d'un lever (27) actionnable de l'extérieur et muni d'un moyen à crochet (28), alors que l'autre support est muni d'une ouverture (26) d'un petit support (25), dans laquelle le moyen à crochet (28) s'insère pour fermer les mâchoires (1-2) alors que, si le moyen à crochet est dégagé de l'ouverture (26), la paire de supports (3-4) et les mâchoires associées (1-2) peuvent être ouverts pour introduire entre ces mâchoires le tissu anatomique à suturer.

9. Instrument selon la revendication 8,
caractérisé en ce que le crochet (28) est muni d'une paire d'encoches (31), la première encoche (30) étant propre à s'engager dans l'ouverture (26) pour assurer une fermeture partielle des mâchoires (1-2) de sorte que le tissu anatomique à suturer peut encore être déplacé pour trouver sa position correcte, et la seconde encoche (31) étant adaptée pour s'engager dans l'ouverture (26) pour assurer une fermeture complète des mâchoires (1-2).

10. Instrument selon la revendication 2,
caractérisé en ce qu'un secteur (32) muni d'un doigt (33) inséré dans un trou (34) prévu dans le tambour (5) peut être tiré extrait du trou (34) afin de permettre au tambour (6) de tourner librement pour déplacer le petit tube de calibrage (15) et avec lui les aiguilles (12-13) le long des mâchoires (1-2).

11. Instrument selon la revendication 4,
caractérisé en ce que la fente périphérique (21) du tambour (5) a un diamètre tel qu'une rotation de 3/4 de tour du bouton (6) corresponde à un mouvement vers l'avant du petit tube de calibrage (15) pour lequel les aiguilles (12-13) sont amenées en saillie à l'extérieur de l'extrémité libre des mâchoires (1-2).
